# EUROPÄISCHE PATENTSCHRIFT

(11) **EP 2 837 342 B1**
(45) Veröffentlichungstag und Bekanntmachung des Hinweises auf die Patenterteilung: **30.09.2015**
(21) Anmeldenummer: 14178740.8
(22) Anmeldetag: 28.07.2014
(51) Int. Cl.: A61B 17/29

(54) **Endoskopisches Instrument**
Endoscopic instrument
Instrument endoscopique

(30) Priorität: 16.08.2013 DE 102013013504
(43) Veröffentlichungstag der Anmeldung: 18.02.2015
(73) Patentinhaber: Karl Storz GmbH & Co. KG, 78532 Tuttlingen (DE)
(72) Erfinder: Abri, Omid, 14129 Berlin (DE); Schrader, Stephan, 14532 Kleinmachnow (DE); Forster, Jonas, 13595 Berlin (DE)
(74) Vertreter: Witte, Weller & Partner Patentanwälte mbB

(56) Entgegenhaltungen:
- EP-A2- 0 646 356
- US-A1- 2003 158 576
- US-A1- 2004 253 079
- US-A1- 2005 187 575

## Beschreibung

Die vorliegende Erfindung betrifft ein endoskopisches Instrument.

Die klassischen Instrumente der minimal-invasiven Chirurgie (MIC) sind vom Griff (proximales Ende) bis hin zum Endeffektor (distales Ende), bei dem es sich insbesondere um einen Greifer oder eine Schere handelt, starr. Das Instrument stellt dem dem Chirurgen üblicherweise zwei Freiheitsgrade zur Verfügung. Zum einen kann der Schaft, und damit der Endeffektor, rotiert werden. Zum anderen kann der Endeffektor geöffnet und geschlossen werden.

Zwar ist es möglich, dass der Chirurg das endoskopische Instrument lateral verlagert, doch stellt dies nur eine geringe Bewegungsfreiheit dar. Diese Bewegungsfreiheit wird umso geringer, je tiefer ein Chirurg das endoskopische Instrument in den Körper eines Patienten einführen muss und je sensibler das umliegende Gewebe ist, das durch eine laterale Verlagerung des Instruments Schaden nehmen könnte.

Vor ähnliche Probleme sieht sich auch der Techniker gestellt, der mit einem endoskopischen Instrument in engen technischen Räumen, wie z.B. in einem Motor, arbeiten muss. Sowohl der Chirurg als auch der Techniker werden durch die geringe Manövrierfähigkeit eingeschränkt und müssen versuchen, über ein erhöhtes Maß an Geschick und Erfahrung diese Einschränkungen wettzumachen.

Im Stand der Technik sind daher verschiedene Lösungen vorgeschlagen worden, um die Beweglichkeit des endoskopischen Instruments an seinem distalen Ende zu verbessern.

US 6,699,235 zeigt ein endoskopisches Instrument, das am distalen Ende in zwei zueinander senkrecht stehenden Ebenen verschwenkt werden kann. Die Vorrichtung wird aber in erster Linie mit einem Endeffektor verwendet, der für seine Betätigung keinen eigenen Freiheitsgrad benötigt, wie z.B. ein Endeffektor zum Kauterisieren.

US 2008/0058861 zeigt ein endoskopisches Instrument mit einem beweglichen distalen Ende, an dem ein Greifer angeordnet ist. Der Aufbau dieses Instruments ist allerdings sehr komplex und gibt dem Chirurgen bei seiner Arbeit keine unmittelbare haptische Rückmeldung.

US 7,121,781 zeigt ein endoskopisches Instrument, dessen distales Ende schwenkbar auf einem Kugelgelenk gelagert ist. Drei Stifte ermöglichen es, das distale Greiferende um zwei Achsen zu verlagern und den Endeffektor zu öffnen und zu schließen. Die Betätigung des Endeffektors muss über eine maschinelle Ansteuerung erfolgen. Zudem fehlt es an einer unmittelbaren haptischen Rückmeldung an den Chirurgen.

US 20030158576 offenbart eine Vorrichtung entsprechend dem Oberbegriff des Anspruchs 1.

Es ist eine Aufgabe, ein verbessertes endoskopisches Instrument bereitzustellen, das dem Chirurgen ausreichende Freiheit bei der Betätigung des Endeffektors gibt, während das endoskopische Instrument im Einsatz ist. Dabei soll das chirurgische Instrument insbesondere mechanisch einfach aufgebaut sein und eine direkte haptische Rückmeldung an den Chirurgen ermöglichen. Dabei soll möglichst auch eine intuitive Bedienung des endoskopischen Instruments gegeben sein, so dass die Bewegungen der Hand des Chirurgen unmittelbar auf den Endeffektor übertragen werden.

Nach einem Aspekt wird die Aufgabe gelöst durch ein endoskopisches Instrument mit:
- einem proximalen Ende,
- einem distalen Ende,
- einer Längsrichtung, entlang derer sich das endoskopische Instrument vom proximalen Ende zum distalen Ende erstreckt,
- einem Endeffektor am distalen Ende, der ein erstes Endeffektorteil und ein zweites Endeffektorteil aufweist,
- einem Lagerelement am distalen Ende, an dem das erste und das zweite Endeffektorteil gelagert sind und an dem Abschnitte einer gewölbten Oberfläche ausgebildet ist,
- einem ersten Schubelement,
- einem zweiten Schubelement, und
- einem dritten Schubelement,
wobei sich die Schubelemente jeweils entlang der Längsrichtung erstrecken und entlang der Längsrichtung relativ zueinander verlagerbar sind,
wobei das erste Schubelement gelenkig mit dem ersten Endeffektorteil gekoppelt ist, das zweite Schubelement gelenkig mit dem zweiten Endeffektorteil gekoppelt ist, das dritte Schubelement gelenkig mit dem ersten Endeffektorteil und dem zweiten Endeffktorteil gekoppelt ist, und
wobei das erste Endeffektorteil eine zumindest abschnittsweise gewölbte erste Lageroberfläche aufweist und das zweite Endeffektorteil eine zumindest abschnittsweise gewölbte zweite Lageroberfläche aufweist und die erste und die zweite Lageroberfläche jeweils auf dem Lagerelement gleitend verschiebbar gehalten sind.

Eine Besonderheit dieses endoskopischen Instruments ist darin zu sehen, dass sich mittels der drei Schubelemente, die insbesondere bis ins proximale Ende des endoskopischen Instruments geführt sind, und der Endeffektorteile, die auf dem Lagerelement angeordnet sind, viele Freiheitsgrade realisieren lassen. So ist es möglich, das erste und das zweite Endeffektorteil, die einen Endeffektor oder zumindest eine Baugruppe des Endeffektors darstellen, relativ zueinander zu verlagern, und zwar bezogen auf das Lagerelement, an dem das erste und das zweite Endeffektorteil gelagert sind.

Des Weiteren ist es möglich, die beiden Endeffektorteile gleichsinnig und gleichmäßig zu verlagern, so dass der Endeffektor in einer ersten Ebene, die von Längsrichtung und einer Hochrichtung aufgespannt ist, verschwenkt werden kann. Außerdem ist es möglich, den Endeffektor in einer zweiten Ebene zu verschwenken, die zu der ersten Ebene senkrecht steht und die durch die Längsrichtung und eine Querrichtung aufgespannt ist.

Eine gleichzeitige Verlagerung des ersten und des zweiten Endeffektorteils in der ersten Ebene und in der zweiten Ebene ist auch möglich. Dadurch kann der Endeffektor in alle Richtungen verschwenkt werden, insbesondere sind auch ovale oder kreisförmige Bewegungen des Endeffektors möglich. Das endoskopische Instrument kann weiterhin entlang seiner Längsrichtung verlagert werden und um eine Längsachse entlang der Längsrichtung gedreht werden. Die Lageroberflächen liegen bevorzugt zumindest abschnittsweise direkt am Lagerelement an.

Es sei an dieser Stelle darauf hingewiesen, dass die bislang eingeführten und später noch hinzutretenden Begriffe "Längsrichtung", "Längsachse", "Querrichtung", "Querachse", "Hochrichtung" und "Hochachse" lediglich einer besseren Orientierung und einem vereinfachten Verständnis des strukturellen Aufbaus dienen. Die Verwendung dieser Begriffe dient nicht dazu, eine bestimmte Beziehung zu einem anderen Bezugssystem, wie z.B. dem Schwerefeld der Erde, herzustellen.

Anhand der verschiedenen Begriffe soll vielmehr aufgezeigt werden, dass bestimmte Erstreckungen des endoskopischen Instruments und Bewegungen des Endeffektors in unterschiedliche Richtungen erfolgen können. Dabei stehen jeweils Längsrichtung und Querrichtung, Längsrichtung und Hochrichtung und Querrichtung und Hochrichtung in einem Winkel zueinander, zeigen also in unterschiedliche Raumrichtungen. Dabei lässt sich das endoskopische Instrument so ausrichten, dass Längsrichtung, Querrichtung und Hochrichtung jeweils paarweise in einem Winkel von mindestens 45°, bevorzugt von mindestens 75°, besonders bevorzugt von mindestens 85° und insbesondere zumindest in etwa senkrecht zueinanderstehen.

Insbesondere soll die Hochrichtung als senkrecht zur Querrichtung verstanden werden. Da das erste und das zweite Endeffektorteil relativ zueinander verschwenkbar sind, müssen eine erste Hochachse des ersten Endeffektorteils und eine zweite Hochachse des zweiten Endeffektorteils nicht in jeder Stellung des Endeffektors zusammenfallen oder parallel sein. Vielmehr wird sich bei der Betätigung des Endeffektorteils, insbesondere bei einem Öffnen und Schließen, ein Winkel zwischen der ersten und der zweiten Hochachse einstellen. Die erste und die zweite Hochachse bleiben aber, abgesehen von extremen Stellungen des Endeffektorteils, jeweils in einem Winkel sowohl zur Querrichtung als auch zur Längsrichtung und sind in ihrer Ausrichtung daher technisch von der Längsrichtung der Querrichtung unterscheidbar.

Die Orientierungshilfe, die mit diesen Bezeichnungen erzielt werden soll, wird in den Figuren nochmals verdeutlicht.

Die Schubelemente erstrecken sich jeweils entlang der Längsrichtung, also entlang der Richtung, in der sich das endoskopische Instrument vom proximalen Ende zum distalen Ende erstreckt.

Es sei darauf hingewiesen, dass die Bezeichnungen "Schubelement", "Schubstange", "Schubkraft", sowohl im Sinne eines positiven Schubs als auch eines negativen Schubs, auch Zug genannt, zu verstehen sind. Die Begriffe werden lediglich als sprachliche Vereinfachung für ein besseres Verständnis der Erfindung verwendet.

Bevorzugt ist die Kopplung zwischen dem ersten Schubelement, dem ersten Endeffektorteil, dem zweiten Schubelement und dem zweiten Endeffektorteil derart ausgestaltet, dass eine Verlagerung des ersten Schubelements und des zweiten Schubelements auf das distale Ende zu das erste und das zweite Endeffektorteil in Schließrichtung aufeinander zubewegt und dass eine Verlagerung des ersten Schubelements und des zweiten Schubelements auf das proximale Ende zu das erste und das zweite Endeffektorteil in Öffnungsrichtung voneinander wegbewegt. Insbesondere wird diese Kopplung unabhängig von einer Bewegung des dritten Schubelements ausgestaltet, so dass die Kopplung bevorzugt ohne eine Verlagerung des dritten Schubelements erfolgt und das dritte Schubelement durch die Verlagerung von erstem und zweitem Schubelement nicht zwangsläufig verlagert wird.

Bevorzugt ist die Kopplung zwischen dem ersten Schubelement, dem ersten Endeffektorteil, dem zweiten Schubelement und dem zweiten Endeffektorteil derart ausgestaltet, dass eine gleichmäßige, aber gegenläufige Verlagerung des ersten Schubelements und des zweiten Schubelements bezogen auf die Längsrichtung ein Verschwenken von erstem und zweitem Endeffektorteil um das Lagerelement bewirkt. Insbesondere wird diese Kopplung unabhängig von einer Bewegung des dritten Schubelements ausgestaltet, so dass die Kopplung bevorzugt ohne eine Verlagerung des dritten Schubelements erfolgt und das dritte Schubelement durch die Verlagerung von erstem und zweitem Schubelement nicht zwangsläufig verlagert wird.

Bevorzugt ist die Kopplung zwischen dem dritten Schubelement und dem ersten und zweiten Endeffktorteil derart ausgestaltet, dass eine Verlagerung des dritten Schubelements bezogen auf die Längsrichtung ein gemeinsames Verschwenken von erstem und zweitem Endeffektorteil jeweils entsprechend um eine erste und eine zweite Hochachse bewirkt, wobei die erste Hochachse und die zweite Hochachse jeweils senkrecht zur Querrichtung stehen. Wenn erste und zweite Hochachse zusammenfallen, so kann dies bevorzugt die Hochrichtung definieren, entlang derer die erste und zweite Hochachse verlaufen.

Bevorzugt ist an einer Seite des endoskopischen Instruments, die der Seite des endoskopischen Instruments mit dem dritten Schubelement gegenüberliegt, außerdem ein viertes Schubelement angeordnet, das bei einem gemeinsamen Verlagern der Endeffektorteile in einer von Hochrichtung und Längsrichtung aufgespannten Ebene gegenläufig zum dritten Schubelement verlagert wird. Das vierte Schubelement ist gelenkig mit dem ersten Endeffektorteil und dem zweiten Endeffktorteil gekoppelt. Das vierte Schubelement verläuft bevorzugt parallel zum dritten Schubelement. Die Erläuterungen zum dritten Schubelement und dessen dritten Gelenk lesen sich entsprechend auf das vierte Schubelement und ein entsprechendes viertes Gelenk. Das vierte Schubelement ermöglicht eine besonders gute Führung der Endeffektorteile. Das vierte Schubelement ist bevorzugt an einem zweiten Endabschnitt eines Schwenkbolzens angeordnet, an dem an einem ersten Endabschnitt die dritte Schubstange angeordnet ist. Der erste Endabschnitt liegt entlang der Längserstreckung des Schwenkbolzens dem zweiten Endabschnitt des Schwenkbolzens gegenüber.

Damit ist die Aufgabe vollständig gelöst.

Bei einer vorteilhaften Ausgestaltung ist das erste Schubelement mittels eines ersten Gelenks gelenkig mit dem ersten Endeffektorteil gekoppelt und/oder das zweite Schubelement mittels eines zweiten Gelenks gelenkig mit dem zweiten Endeffektorteil gekoppelt und/oder das dritte Schubelement mittels eines dritten Gelenks gelenkig mit dem ersten Endeffektorteil und dem zweiten Endeffktorteil gekoppelt.

Diese Ausgestaltung ermöglicht eine gute Übertragung der Schub- bzw. Zugkraft von den Schubelementen auf die Endeffektorteile. Außerdem können die Endeffektorteile präzise verlagert werden. Es ist vorteilhaft, wenn alle gelenkigen Lagerungen jeweils als Gelenk ausgeführt sind. Bei einer bevorzugten Ausführungsform wird ein Festkörpergelenk verwendet, insbesondere aus Federstahl oder Formgedächtnis-Legierungen, bevorzugt Nitinol (Nickel-Titan-Legierung). Bei einer anderen bevorzugten Ausführungsform wird ein elastisches Bauteil, z.B. aus Gummi oder Kunststoff, verwendet.

Bei einer weiteren vorteilhaften Ausgestaltung sind die gelenkige Kopplung des ersten Schubelements und die gelenkige Kopplung des zweiten Schubelements derart ausgebildet, dass bei einer Verlagerung des ersten Schubelements und/oder des zweiten Schubelements das erste Endeffektorteil und das zweite Endeffektorteil relativ zueinander in einer ersten Schwenkebene um eine erste Schwenkachse verschwenkbar sind.

Diese Ausgestaltung ermöglicht auf einfache Weise die Verlagerung des ersten Endeffektorteils und des zweiten Endeffektorteils in der ersten Schwenkebene. Dabei können das erste und das zweite Endeffektorteil gleichmäßig verschwenkt werden, um ein Verschwenken des Endeffektors zu erzielen. Es ist aber auch möglich, das erste und zweite Endeffektorteil unterschiedlich zu verschwenken, um ein Öffnen oder ein Schließen des Endeffektors zu erzielen. Gemäß der zuvor erläuterten Orientierungshilfe liegt die erste Schwenkebene insbesondere in der Ebene, die von Längsrichtung und Hochrichtung aufgespannt ist.

Bei einer weiteren vorteilhaften Ausgestaltung ist die gelenkige Kopplung des dritten Schubelements derart ausgebildet, dass bei einer Verlagerung des dritten Schubelements das erste Endeffektorteil und das zweite Endeffektorteil gemeinsam in einer zweiten Schwenkebene verlagerbar sind.

Diese Ausgestaltung ermöglicht es auf einfache Weise, den Endeffektor in einer zweiten Dimension zu verlagern. Dabei ist die gelenkige Kopplung des dritten Schubelements bevorzugt derart ausgestaltet, dass eine relative Position der beiden Effektorteile zueinander unverändert bleibt. Im Hinblick auf die zuvor eingeführte Orientierungshilfe liegt die zweite Schwenkebene insbesondere in der Ebene, die von Längsrichtung und Querrichtung aufgespannt ist. Eine zweite Schwenkachse, um die sich der Endeffektor verschwenkt, verläuft bevorzugt entlang der Hochrichtung und führt dabei insbesondere durch das erste Gelenk und das zweite Gelenk. Da das Instrument insgesamt um die Längsrichtung gedreht werden kann, kann der Endeffektor daher in einer nahezu beliebigen Position und Orientierung geöffnet und geschlossen werden.

Bei einer weiteren vorteilhaften Ausgestaltung ist das dritte Schubelement entlang einer ersten Schwenkachse mit dem ersten Endeffektorteil und mit dem zweiten Endeffektorteil gekoppelt.

Bei der ersten Schwenkachse handelt es sich bevorzugt um die Achse, um die die Endeffektorteile relativ zueinander verschwenkt werden können. Dadurch, dass der Anlenkpunkt des dritten Schubelements entlang der ersten Schwenkachse liegt, ist der Aufbau besonders einfach und zuverlässig. Da bei einer Betätigung des dritten Schubelements die Schwenkachse des ersten und zweiten Endeffektorteils verlagert wird, wird durch eine Betätigung des dritten Schubelements insbesondere keine oder nur eine geringe Kraft auf die Endeffektorteile ausgeübt, die die relative Position der Endeffektorteile zueinander verändert.

Bei einer weiteren vorteilhaften Ausgestaltung ist das erste Gelenk derart ausgebildet, dass sich das erste Endeffektorteil relativ zum ersten Schubelement um eine erste Drehachse und um eine weitere erste Drehachse verlagern kann, wobei die erste Drehachse und die weitere erste Drehachse in einem ersten Winkel zueinander stehen, insbesondere zumindest in etwa in einem rechten Winkel.

Diese Ausgestaltung ermöglicht eine besonders präzise Steuerung des ersten Endeffektorteils bei einer Betätigung des ersten Schubelements und erlaubt gleichzeitig die Verlagerung des ersten Schubelements in der zweiten Schwenkebene. Dabei ist es besonders vorteilhaft, wenn das erste Gelenk so ausgebildet ist, dass sich das erste Endeffektorteil relativ zum ersten Schubelement ausschließlich um die erste Drehachse und um die weitere erste Drehachse verlagern kann. Dieselben Überlegungen und Vorteile treffen auch auf das zweite Gelenk im Hinblick auf die Verbindung zwischen dem zweiten Endeffektorteil und dem zweiten Schubelement zu.

Bei einer weiteren vorteilhaften Ausgestaltung bildet die erste Schwenkachse eine Längsachse eines Schwenkbolzens, an dem das erste Endeffektorteil und das zweite Endeffektorteil gelagert sind, wobei insbesondere der Schwenkbolzen an einer ersten Seite und an einer zweiten, der ersten Seite gegenüberliegenden Seite aus dem Lagerelement herausragt.

Diese Ausgestaltung ist einfach zu realisieren und ermöglicht eine besonders präzise Führung und Positionierung der beiden Endeffektorteile zueinander. Die Verbindung der Endeffektorteile mit dem Schwenkbolzen lässt sich besonders einfach realisieren, wenn der Schwenkbolzen an zwei Seiten aus dem Lagerelement herausragt. Insbesondere kann dann außerdem der Anlenkpunkt des dritten Schubelements konstruktiv einfach am Schwenkbolzen ausgebildet werden.

Bei einer weiteren vorteilhaften Ausgestaltung ist der Schwenkbolzen durch eine Ausnehmung des Lagerelements geführt und/oder ist das Lagerelement zumindest abschnittsweise kugelsegmentförmig ausgebildet.

Die Ausnehmung im Lagerelement ermöglicht es auf einfache Weise, den Schwenkbolzen und die Endeffektorteile zu positionieren. Dabei ist es insbesondere möglich, den Schwenkbolzen lose durch das Lagerelement zu führen. Bei einer bevorzugten Ausführungsform liegt der Schwenkbolzen am Lagerelement an und kann durch Kippen auf einer gekrümmten Oberfläche die Verlagerung des Endeffektors in der zweiten Schwenkebene sicherstellen. Bei einer anderen bevorzugten Ausführungsform ist der Schwenkbolzen verschwenkbar auf einem Schwenkstift gehalten, der insbesondere mittig innerhalb des Lagerelements angeordnet ist. Wenn das Lagerelement zumindest abschnittsweise kugelsegmentförmig ausgebildet ist, können die Endeffektorteile besonders präzise und gleichmäßig verlagert werden.

Bei einer weiteren bevorzugten Ausgestaltung liegen das erste Schubelement und das zweite Schubelement in einer ersten Schwenkebene und liegt das dritte Schubelement in einer zur ersten Schwenkebene senkrechten zweiten Schubebene.

Diese Ausgestaltung ermöglicht es auf einfache Weise, den Endeffektor in zwei verschiedene Raumrichtungen zu verlagern, die insbesondere senkrecht zueinander stehen. Die erste Schwenkebene wird dabei insbesondere durch die Längsrichtung und die Hochrichtung gebildet. Die zweite Schwenkebene wird dabei insbesondere durch die Längsrichtung und die Querrichtung gebildet. Die zweite Schwenkebene liegt bevorzugt zumindest in etwa mittig zwischen dem ersten und dem zweiten Schubelement und/oder mittig zwischen dem ersten und dem zweiten Gelenkpunkt.

Bei einer weiteren vorteilhaften Ausgestaltung ist das Lagerelement bezogen auf das proximale Ende ortsfest angeordnet und/oder ist das Lagerelement so angeordnet, dass eine Verlagerung eines der Schubelemente immer zu einer Verlagerung des Schubelements relativ zum Lagerelement führt.

Das Lagerelement kann bezogen auf das proximale Ende verlagerbar oder verschiebbar angeordnet sein, insbesondere auf einem Halter, zum Beispiel einem Halterohr oder einem Haltestab. Wird der Halter dann zum Beispiel entlang der Längsrichtung verlagert, während des erste und das zweite Schubelement ruhen, so kann eine Öffnung oder ein Schließen des Endeffektors bewirkt werden. Es wird aber als vorteilhaft angesehen, dass das Lagerelement ortsfest, bezogen auf das proximale Ende, angeordnet ist. Dafür ist das Lagerelement insbesondere starr mit dem proximalen Ende des endoskopischen Instruments verbunden. Diese technische Wirkung erzielt man auch, wenn jede Verlagerung eines der Schubelemente immer zu einer Verlagerung eben dieses Schubelements relativ zum Lagerelement führt. Es ist dabei unerwünscht, dass das Lagerelement, gegebenenfalls in Verbindung mit dem Halter, eine eigene Bewegung vollführt, während eines der Schubelemente betätigt wird. Dabei kann das Lagerelement bei einer, bezogen auf das proximale Ende, ortsfesten Positionierung einen räumlichen Referenzpunkt für den Endeffektor bilden, sodass besonders präzise gearbeitet werden kann. Die Verbindung des Lagerelements mit dem proximalen Ende wird insbesondere dadurch erzielt, dass das Lagerelement starr mit einem Kanal verbunden ist, in dem die Schubelemente geführt sind, wobei der Kanal starr mit dem proximalen Ende verbunden ist.

Bei einer weiteren vorteilhaften Ausgestaltung weist das erste Endeffektorteil eine erste Ausnehmung und eine weitere erste Ausnehmung auf, mit denen das erste Endeffektorteil an einem Schwenkbolzen gehalten ist.

Diese Ausgestaltung ermöglicht eine besonders präzise Verlagerung des ersten Endeffektorteils. Dieselben Überlegungen und Vorteile treffen bevorzugt auch auf das zweite Endeffektorteil zu, das dann mit einer zweiten Ausnehmung und einer weiteren zweiten Ausnehmung an dem genannten Schwenkbolzen gehalten ist.

Bei einer weiteren vorteilhaften Ausgestaltung hat die erste Lageroberfläche zumindest in etwa die Form eines Abschnitts einer Kugeloberfläche, wobei sich der Abschnitt ergibt, indem eine Kugel von zwei nicht-parallelen Ebenen geschnitten wird, die in einem Ebenenwinkel zueinander stehen, wobei der Kugelmittelpunkt in beiden Ebenen liegt.

Diese Ausgestaltung ermöglicht eine besonders genaue und gleichmäßige Verlagerung des ersten Endeffektorteils relativ zum Lagerelement und auch relativ zum zweiten Endeffektorteil. Dieselben Überlegungen und Vorteile treffen in entsprechender Weise auf die zweite Lageroberfläche zu. In den Ausführungsbeispielen werden in diesem Zusammenhang eine erste und eine zweite Lageroberfläche gezeigt, die zumindest in etwa die Form einer Viertelkugel haben, wo die genannten zwei nicht-parallelen Ebenen also in einem Winkel von zumindest ungefähr 90° stehen.

Bei einer weiteren vorteilhaften Ausgestaltung der Erfindung sind alle Schubelemente parallel zueinander angeordnet.

Diese Ausgestaltung ist konstruktiv einfach und zuverlässig. Außerdem kann der Durchmesser des endoskopischen Instruments dadurch gering gehalten werden.

Bei einer weiteren vorteilhaften Ausgestaltung der Erfindung sind die Schubelemente als Schubstangen ausgestaltet, insbesondere vollständig geradlinig ausgestaltet.

Auch diese Ausgestaltung ist konstruktiv einfach und zuverlässig.

Bei einer weiteren vorteilhaften Ausgestaltung der Erfindung ist das erste Endeffektorteil als erstes Greifzangenteil und das zweite Endeffektorteil als zweites Greifzangenteil ausgebildet oder ist das erste Endeffektorteil als erstes Scherenteil und das zweite Endeffektorteil als zweites Scherenteil ausgebildet.

Diese Ausgestaltungen sind besonders vorteilhaft, da dem Benutzer auf einfache Weise alle erforderlichen Freiheitsgrade zur Verfügung gestellt werden können. Grundsätzlich können aber auch andere Endeffektoren verwendet werden, wie z.B. Zangen, Scheren, Koagulationsnadeln oder Fluidabsaugungen, sowie Maulteile.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: ein Ausführungsbeispiel eines endoskopischen Instruments in Seitenansicht;
- Fig. 2: den vorderen Teil des Ausführungsbeispiels in der Draufsicht ohne das Lagerelement;
- Fig. 3: den vorderen Teil des Ausführungsbeispiels in der Seitenansicht ohne das Lagerelement;
- Fig. 4: das Ausführungsbeispiel in einer Ansicht auf die Lageroberflächen der Endeffektorteile;
- Fig. 5: eine Explosionsansicht der Endeffektorteile und des Schwenkbolzens des Ausführungsbeispiels;

- Fig. 6: den vorderen Teil des Ausführungsbeispiels in perspektivischer Ansicht;
- Fig. 7: die Darstellung gemäß Fig. 6 ohne das erste Endeffktorteil;
- Fig. 8: die Darstellung gemäß Fig. 7 ohne das zweite Endeffktorteil;
- Fig. 9: die Darstellung gemäß Fig. 8 ohne das Lagerelement; und
- Fig. 10: das Lagerelement des Ausführungsbeispiels.

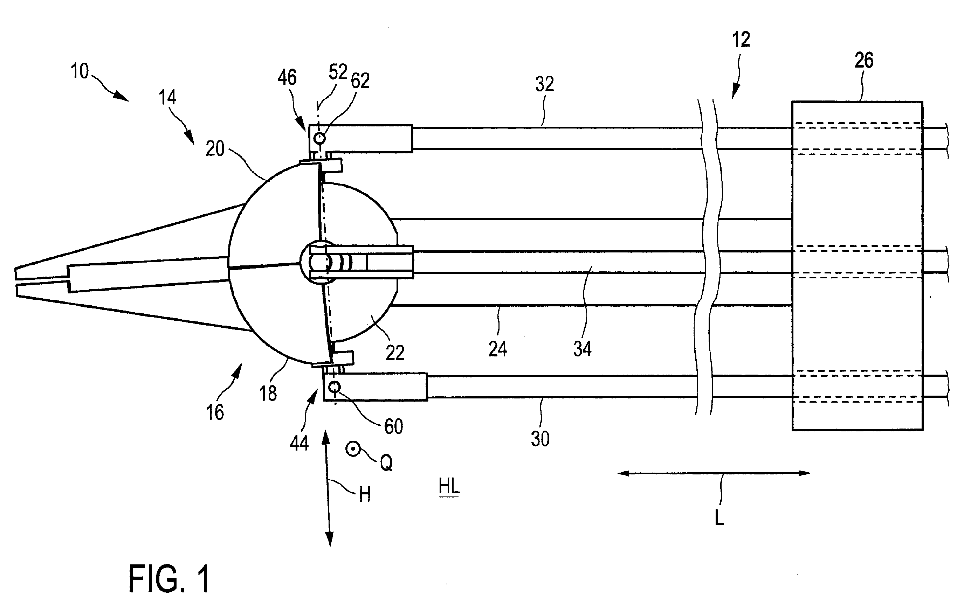

Fig. 1 zeigt ein erstes Ausführungsbeispiel eines endoskopischen Instruments 10 mit einem proximalen Ende 12 und einem distalen Ende 14. Als Orientierung sind eine Längsrichtung L, entlang derer sich das endoskopische Instrument 10 vom proximalen Ende 12 zum distalen Ende 14 erstreckt, eine Querrichtung Q, die quer zur Längsrichtung L verläuft, und eine Hochrichtung H eingezeichnet, wobei die Hochrichtung H sowohl quer zur Längsrichtung L als auch quer zur Querrichtung Q verläuft.

Am distalen Ende 14 weist das endoskopische Instrument 10 einen Endeffektor 16 auf, der ein erstes Endeffektorteil 18 und ein zweites Endeffektorteil 20 aufweist. Ferner ist am distalen Ende 14 ein Lagerelement 22 angeordnet, an dem das erste und das zweite Endeffektorteil 18, 20 gelagert sind. Mit anderen Worten stützen sich die Endeffektorteile 18, 20 bei dieser Ausführungsform auf dem Lagerelement 22 ab.

Das Instrument 10 weist ein erstes Schubelement 30, ein zweites Schubelement 32 und ein drittes Schubelement 34. Die Schubelemente 30, 32, 34 erstrecken sich jeweils entlang der Längsrichtung L und sind entlang der Längsrichtung L relativ zueinander verlagerbar.

Das erste Schubelement 30 ist gelenkig mit dem ersten Endeffektorteil 18 gekoppelt. Das zweite Schubelement 32 ist gelenkig mit dem zweiten Endeffektorteil 20 gekoppelt. Das dritte Schubelement 34 ist gelenkig mit dem ersten Endeffektorteil 18 und mit dem zweiten Endeffektorteil 20 gekoppelt.

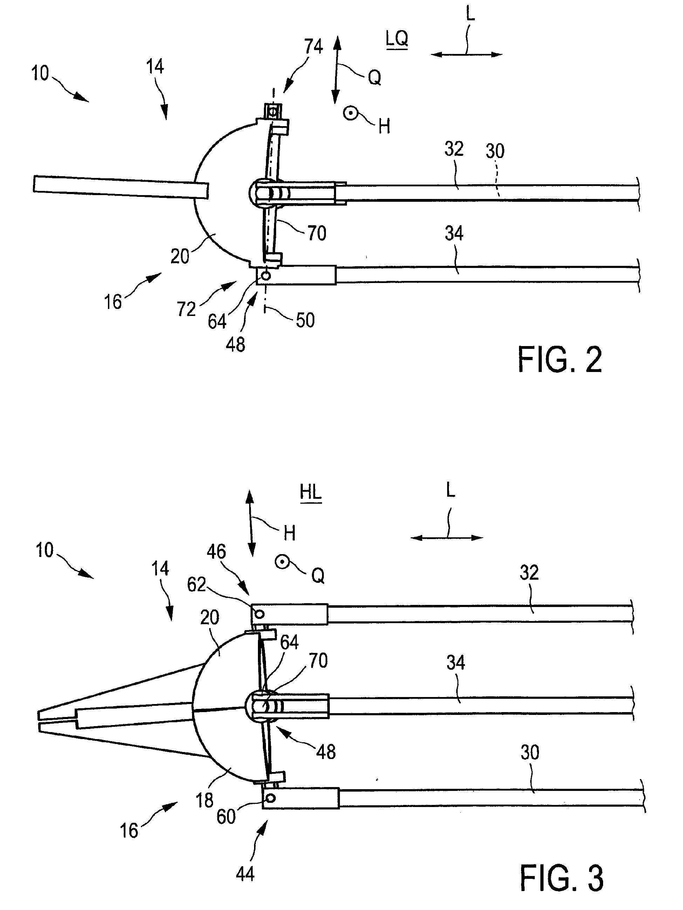

Das erste Schubelement 30 ist mittels eines ersten Gelenks 44 gelenkig am ersten Endeffektorteil 18 gelagert. Außerdem ist auch das zweite Schubelement 32 mittels eines zweiten Gelenks 46 gelenkig am zweiten Endeffektorteil 20 gelagert. Es ist zwar grundsätzlich möglich, zumindest eines der Schubelemente 30, 32 starr mit dem jeweiligen Endeffektorteil 18, 20 zu koppeln, doch wird ein besonderer Vorteil darin gesehen, dass beide Endeffektorteile 18, 20 individuell bewegt werden können. Ferner ist auch das dritte Schubelement 34 mittels eines dritten Gelenks 48, siehe Fig. 2, gelenkig am ersten und am zweiten Endeffektorteil 18, 20 gelagert.

Bei der hier gezeigten Ausführungsform ist das Lagerelement 22 auf einem Halter 24 angeordnet. Der Halter 24 ist an einem Halteblock 26 befestigt, wobei der Halteblock 26 relativ zum proximalen Ende 12 ortsfest angeordnet ist. Anhand der gestrichelten Linien ist angedeutet, dass sich die Schubelemente 30, 32, 34 relativ zum Halteblock 26 und damit relativ zum Halter 24 und zum Lagerelement 22 verlagern können.

Der Endeffektor 16 ist in einer ersten Schwenkebene HL verschwenkbar, die durch Längsrichtung L und Hochrichtung H aufgespannt ist. Der Endeffektor 16 ist außerdem in einer zweiten Schwenkebene LQ verschwenkbar, die von der Längsrichtung L und der Querrichtung Q aufgespannt ist, siehe Fig. 2.

Das Verschwenken des Endeffektors 16 in der ersten Schwenkebene HL findet hier um eine erste Schwenkachse 50 statt, siehe Fig. 2, die hier mit der Querrichtung Q zusammenfällt. Das Verschwenken des Endeffektors 16 in der zweiten Schwenkebene LQ findet um eine zweite Schwenkachse 52 statt, die hier mit der Hochrichtung H zusammenfällt. Das erste Schubelement 30 ist an einem ersten Anlenkpunkt 60 mit dem ersten Endeffektorteil 18 gekoppelt, das zweite Schubelement 32 ist an einem zweiten Anlenkpunkt 62 mit dem zweiten Endeffektorteil 20 gekoppelt, und das dritte Schubelement 34 ist an einem dritten Anlenkpunkt 64 mit dem ersten und dem zweiten Endeffektorteil 18, 20 gekoppelt, siehe Fig. 2.

Fig. 2 zeigt den vorderen Teil des Ausführungsbeispiels in der Draufsicht. In dieser Darstellung wurden das proximale Ende 12, das Lagerelement 22 und der Halter 24 weggelassen.

Es ist zu erkennen, dass die Schwenkachse 50 eine Längsachse eines Schwenkbolzens 70 bildet, an dem das dritte Schubelement 34 über den Anlenkpunkt 64 angelenkt ist. Da sowohl das erste Endeffektorteil 18 als auch das zweite Endeffektorteil 20 an dem Schwenkbolzen 70 angeordnet sind, ist auf diese Weise das dritte Schubelement 34 mit dem ersten und dem zweiten Endeffektorteil 18, 20 gekoppelt. Es ist zu erkennen, dass der Schwenkbolzen 70 sowohl an einer ersten Seite 72 als auch an einer zweiten Seite 74 über das Lagerelement 22, siehe Fig. 1, herausragt.

Das erste Schubelement 30 und das zweite Schubelement 32 liegen in einer ersten Schwenkebene HL. Das dritte Schubelement 34 liegt in der zur ersten Schwenkebene HL senkrechten zweiten Schwenkebene LQ.

Die Schubelemente 30, 32, 34 sind hier parallel zueinander angeordnet. Sie sind hier als Schubstangen ausgestaltet und dabei insbesondere vollständig geradlinig ausgestaltet. Das erste Endeffektorteil 18 ist als erstes Greifzangenteil und das zweite Endeffektorteil 20 als zweites Greifzangenteil ausgebildet.

Fig. 3 zeigt den vorderen Teil des Ausführungsbeispiels in der Seitenansicht, hier allerdings - im Vergleich zur Fig. 1 - ohne das Lagerelement 22 und den Halter 24. Es gelten die Ausführungen, die bereits zu den Figuren 1 und 2 gemacht wurden.

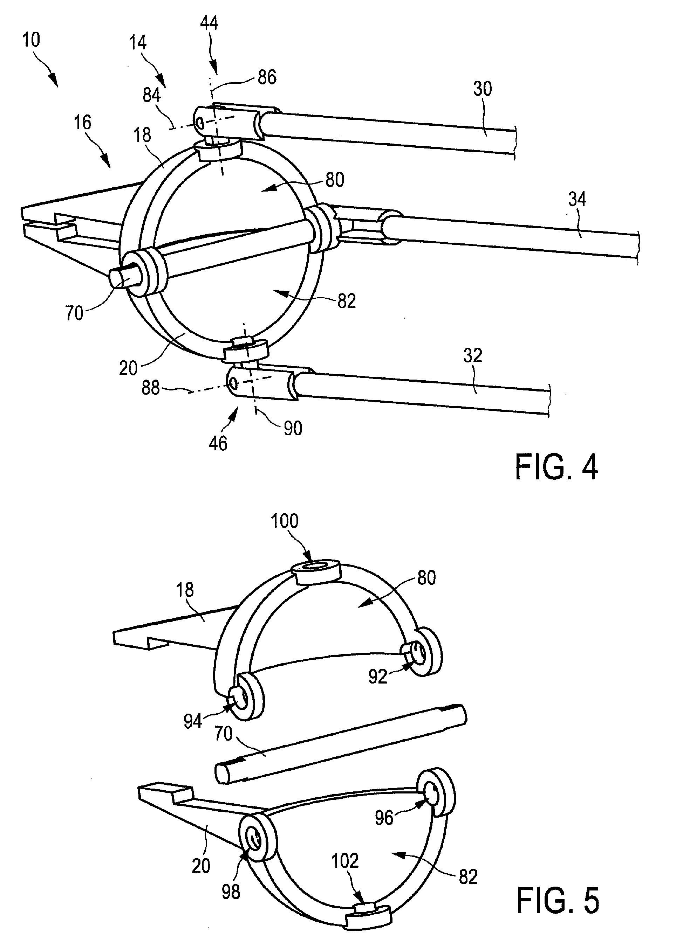

Fig. 4 zeigt das Ausführungsbeispiel in einer Ansicht auf die Lageroberflächen 80, 82 der Endeffektorteile 18, 20. Die erste Lageroberfläche 80 und die zweite Lageroberfläche 82 sind zumindest abschnittsweise gewölbt und haben hier jeweils zumindest in etwa die Form einer Viertelkugel. Es wird als vorteilhaft angesehen, wenn die Lageroberflächen 80, 82 geschlossen sind. Bei bestimmten Ausführungsformen können die Lageroberflächen 80, 82 aber auch Ausnehmungen und/oder Vorsprünge und/oder Einbuchtungen haben.

Das erste Gelenk 44 ist derart ausgebildet, dass sich das erste Endeffektorteil 18 relativ zum ersten Schubelement 30 um eine erste Drehachse 84 und um eine weitere erste Drehachse 86 verlagern kann. Das erste Gelenk 44 ist insbesondere so ausgebildet, dass sich das erste Endeffektorteil 18 relativ zum ersten Schubelement 30 nur um die erste und die weitere erste Drehachse 84, 86 verlagern kann.

Das zweite Gelenk 46 ist derart ausgebildet, dass sich das zweite Endeffektorteil 20 relativ zum zweiten Schubelement 32 um eine zweite Drehachse 88 und um eine weitere zweite Drehachse 90 verlagern kann. Das zweite Gelenk 46 ist insbesondere so ausgebildet, dass sich das zweite Endeffektorteil 20 relativ zum zweiten Schubelement 32 nur um die zweite und die weitere zweite Drehachse 88, 90 verlagern kann. Bei einer weiteren bevorzugten Ausgestaltung ist am freien Ende des Schwenkbolzens 70 (hier im Vordergrund) ein viertes Schubelement (nicht gezeigt) angeordnet, das bei einem gemeinsamen Verlagern der Endeffektorteile 18, 20 in der HL-Ebene gegenläufig zum dritten Schubelement 34 verlagert wird. Das vierte Schubelement verläuft bevorzugt parallel zum dritten Schubelement 34. Die Verbindung des vierten Schubelements mit dem Schwenkbolzen 70 ist bevorzugt in gleicher Weise ausgestaltet, wie die Verbindung des dritten Schubelements 34 mit dem Schwenkbolzen 70.

Fig. 5 zeigt eine Explosionsansicht der Endeffektorteile 18, 20 und des Schwenkbolzens 70. Das erste Endeffektorteil 18 weist eine erste Ausnehmung 92 und eine weitere erste Ausnehmung 94 auf, mit denen das erste Endeffektorteil 18 an dem Schwenkbolzen 70 gehalten ist. Das zweite Endeffektorteil 20 weist eine zweite Ausnehmung 96 und eine weitere zweite Ausnehmung 98 auf, mit denen das zweite Endeffektorteil 20 an dem Schwenkbolzen 70 gehalten ist. Es sind ferner eine zusätzliche erste Ausnehmung 100 und eine zusätzliche zweite Ausnehmung 102 zu erkennen, über die das erste Schubelement 30 bzw. das zweite Schubelement 32 mit dem jeweiligen Endeffektorteil 18, 20 gekoppelt ist.

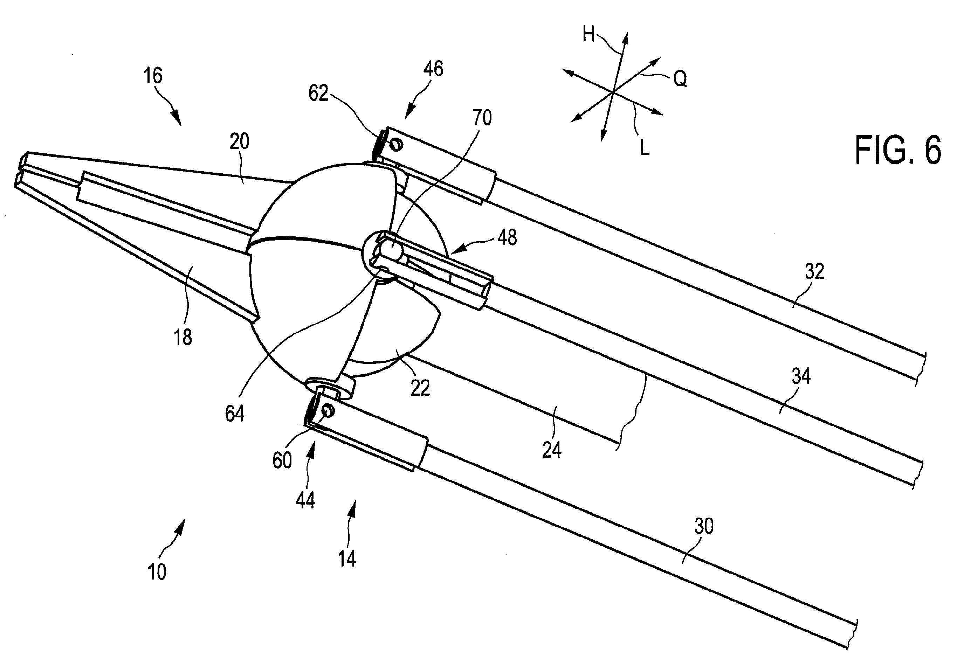

Fig. 6 zeigt den vorderen Teil des Ausführungsbeispiels in perspektivischer Ansicht. Es gelten weiterhin alle zuvor gemachten Ausführungen.

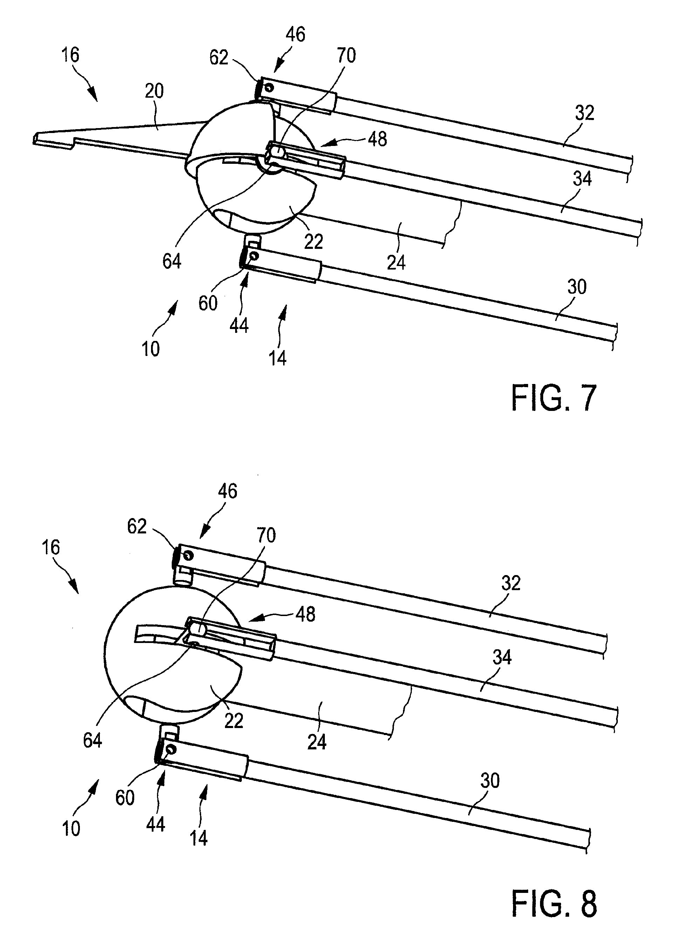

Fig. 7 zeigt die Darstellung gemäß Fig. 6, allerdings ohne das erste Endeffektorteil 18. Diese Darstellung ermöglicht eine Sicht auf das Lagerelement 22. Es gelten weiterhin alle bereits zuvor gemachten Aussagen.

Fig. 8 zeigt die Darstellung gemäß Fig. 7, allerdings ohne das zweite Endeffektorteil 20. Das Lagerelement 22 ist nun noch besser zu erkennen. Es gelten weiterhin alle bereits zuvor gemachten Aussagen.

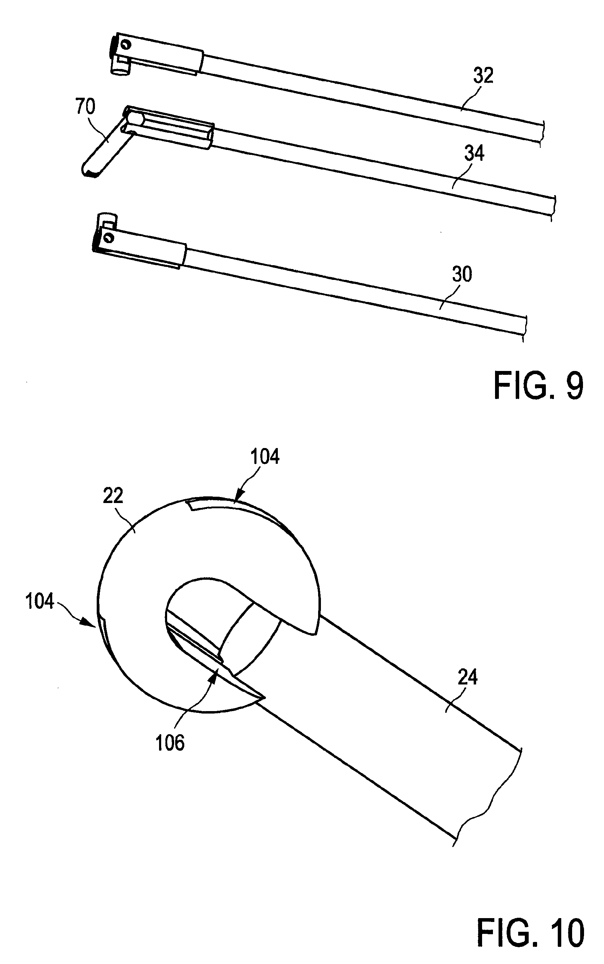

Fig. 9 zeigt die Darstellung gemäß Fig. 8, allerdings ohne das Lagerelement 22 und den Halter 24. Es sind nun besonders gut die Punkte zu erkennen, an denen die Endeffektorteile 18, 20 über die jeweiligen Gelenke 44, 46, 48 mit den Schubelementen 30, 32, 34 gekoppelt sind.

Fig. 10 zeigt das Lagerelement 22 mit dem Halter 24 in einer Detaildarstellung. Es ist die Ausnehmung 104 zu erkennen, durch die der Schwenkbolzen 70 durch das Lagerelement 22 geführt ist. Außerdem ist zu erkennen, dass das Lagerelement 22 zumindest abschnittsweise kugelsegmentförmig ausgebildet ist. Der Begriff Kugelsegment soll dabei so verstanden werden, dass zumindest Teile einer Kugel erkennbar sind, die Kugel aber nicht vollständig sein muss und die Kugeloberfläche Ausnehmungen aufweisen darf. Das Lagerelement 22 hat außerdem eine weitere Ausnehmung 106.

Damit wurde insgesamt ein besonders vorteilhaftes endoskopisches Instrument 10 aufgezeigt, das mit einer mechanisch zu realisierenden Lösung dem Chirurgen viele Freiheitsgrade für die Betätigung des Endeffektors bietet und dabei auch eine intuitive Bedienbarkeit ermöglicht.

## Patentansprüche

1. Endoskopisches Instrument (10) mit
- einem proximalen Ende (12),
- einem distalen Ende (14),
- einer Längsrichtung (L), entlang derer sich das endoskopische Instrument (10) vom proximalen Ende (12) zum distalen Ende (14) erstreckt,
- einem Endeffektor (16) am distalen Ende (14), der ein erstes Endeffektorteil (18) und ein zweites Endeffektorteil (20) aufweist,
- einem Lagerelement (22) am distalen Ende (14), an dem das erste und das zweite Endeffektorteil (18, 20) gelagert sind und an dem Abschnitte einer gewölbten Oberfläche ausgebildet ist,
- einem ersten Schubelement (30),
- einem zweiten Schubelement (32), und
- einem dritten Schubelement (34),
wobei sich die Schubelemente (30, 32, 34) jeweils entlang der Längsrichtung (L) erstrecken und entlang der Längsrichtung (L) relativ zueinander verlagerbar sind,
wobei das erste Schubelement (30) gelenkig mit dem ersten Endeffektorteil (18) gekoppelt ist, das zweite Schubelement (32) gelenkig mit dem zweiten Endeffektorteil (20) gekoppelt ist, das dritte Schubelement (34) gelenkig mit dem ersten Endeffektorteil (18) und dem zweiten Endeffktorteil (20) gekoppelt ist, **dadurch gekennzeichnet, dass**
das erste Endeffektorteil (18) eine zumindest abschnittsweise gewölbte erste Lageroberfläche (80) aufweist und das zweite Endeffektorteil (20) eine zumindest abschnittsweise gewölbte zweite Lageroberfläche (82) aufweist und die erste und die zweite Lageroberfläche (80, 82) jeweils auf dem Lagerelement (22) gleitend verschiebbar gehalten sind.

2. Endoskopisches Instrument nach Anspruch 1, wobei das erste Schubelement (30) mittels eines ersten Gelenks (44) gelenkig mit dem ersten Endeffektorteil (18) gekoppelt ist und/oder das zweite Schubelement (32) mittels eines zweiten Gelenks (46) gelenkig mit dem zweiten Endeffektorteil (20) gekoppelt ist und/oder das dritte Schubelement (32) mittels eines dritten Gelenks (48) gelenkig mit dem ersten Endeffektorteil (18) und dem zweiten Endeffktorteil (20) gekoppelt ist.

3. Endoskopisches Instrument nach einem der vorhergehenden Ansprüche, wobei die gelenkige Kopplung des ersten Schubelements (30) und die gelenkige Kopplung des zweiten Schubelements (32) derart ausgebildet sind, dass bei einer Verlagerung des ersten Schubelements (30) und/oder des zweiten Schubelements (32) das erste Endeffektorteil (18) und das zweite Endeffektorteil (20) relativ zueinander in einer ersten Schwenkebene (HL) um eine erste Schwenkachse (50) verschwenkbar sind.

4. Endoskopisches Instrument nach einem der vorhergehenden Ansprüche, wobei die gelenkige Kopplung des dritten Schubelements (34) derart ausgebildet ist, dass bei einer Verlagerung des dritten Schubelements (34) das erste Endeffektorteil (18) und das zweite Endeffektorteil (20) gemeinsam in einer zweiten Schwenkebene (LQ) verlagerbar sind.

5. Endoskopisches Instrument nach einem der vorhergehenden Ansprüche, wobei das dritte Schubelement (34) entlang einer ersten Schwenkachse (50) mit dem ersten Endeffektorteil (18) und dem zweiten Endeffktorteil (20) gekoppelt ist.

6. Endoskopisches Instrument nach einem der Ansprüche 2 bis 5, wobei das erste Gelenk (44) derart ausgebildet ist, dass sich das erste Endeffektorteil (18) relativ zum ersten Schubelement (30) um eine erste Drehachse (84) und um eine weitere erste Drehachse (86) verlagern kann, wobei die erste Drehachse (84) und die weitere erste Drehachse (86) in einem ersten Winkel zueinander stehen, insbesondere zumindest in etwa im einem rechten Winkel.

7. Endoskopisches Instrument nach einem der Ansprüche 3 bis 6, wobei die erste Schwenkachse (50) eine Längsachse eines Schwenkbolzens (70) bildet, an dem das erste Endeffektorteil (18) und das zweite Endeffektorteil (20) gelagert sind, wobei insbesondere der Schwenkbolzen (70) an einer ersten Seite (72) und an einer zweiten, der ersten Seite gegenüberliegenden Seite (74) aus dem Lagerelement (22) herausragt.

8. Endoskopisches Instrument nach Anspruch 7, wobei der Schwenkbolzen (70) durch eine Ausnehmung (104) des Lagerelements (22) geführt ist und/oder das Lagerelement (22) zumindest abschnittsweise kugelsegmentförmig ausgebildet ist.

9. Endoskopisches Instrument nach einem der vorhergehenden Ansprüche, wobei das erste Schubelement (30) und das zweite Schubelement (32) in einer ersten Schwenkebene (HL) liegen und das dritte Schubelement (34) in einer zur ersten Schwenkebene (HL) senkrechten zweiten Schwenkebene (LQ) liegt.

10. Endoskopisches Instrument nach einem der vorhergehenden Ansprüche, wobei das Lagerelement (22) bezogen auf das proximale Ende (12) ortsfest angeordnet ist und/oder das Lagerelement (22) so angeordnet ist, dass eine Verlagerung eines der Schubelemente (30, 32, 34) immerzu einer Verlagerung des Schubelements (30, 32, 34) relativ zum Lagerelement (22) führt.

11. Endoskopisches Instrument nach einem der vorhergehenden Ansprüche, wobei das erste Endeffektorteil (18) eine erste Ausnehmung (92) und eine weitere erste Ausnehmung (94) aufweist, mit denen das erste Endeffektorteil (18) an einem Schwenkbolzen (70) gehalten ist.

12. Endoskopisches Instrument nach einem der vorhergehenden Ansprüche, wobei die erste Lageroberfläche (80) zumindest in etwa die Form eines Abschnitts einer Kugeloberfläche hat, wobei sich der Abschnitt ergibt, indem eine Kugel von zwei nicht-parallelen Ebenen geschnitten wird, die in einem Ebenenwinkel zueinanderstehen, wobei der Kugelmittelpunkt in beiden Ebenen liegt.

13. Endoskopisches Instrument nach einem der vorhergehenden Ansprüche, wobei alle Schubelemente (30, 32, 34) parallel zueinander angeordnet sind.

14. Endoskopisches Instrument nach einem der vorhergehenden Ansprüche, wobei die Schubelemente (30, 32, 34) als Schubstangen ausgestaltet sind, insbesondere vollständig geradlinig ausgestaltet sind.

15. Endoskopisches Instrument nach einem der vorhergehenden Ansprüche, wobei das erste Endeffektorteil (18) als erstes Greifzangenteil und das zweite Endeffektorteil (20) als zweites Greifzangenteil ausgebildet ist oder wobei das erste Endeffektorteil (18) als erstes Scherenteil und das zweite Endeffektorteil (20) als zweites Scherenteil ausgebildet ist.

## Claims

1. Endoscopic instrument (10) having
- a proximal end (12),
- a distal end (14),
- a longitudinal direction (L) along which the endoscopic instrument (10) extends from the proximal end (12) to the distal end (14),
- an end effector (16) at the distal end (14), which has a first end effector part (18) and a second end effector part (20),
- a bearing element (22) at the distal end (14), on which the first and second end effector parts (18, 20) are arranged and on which portions of a curved surface are formed,
- a first thrust element (30),
- a second thrust element (32), and
- a third thrust element (34),
wherein the thrust elements (30, 32, 34) each extend along the longitudinal direction (L) and are displaceable relative to one another along the longitudinal direction (L),
wherein the first thrust element (30) is articulatedly coupled to the first end effector part (18), the second thrust element (32) is articulatedly coupled to the second end effector part (20), the third thrust element (34) is articulatedly coupled to the first end effector part (18) and the second end effector part (20),
**characterized in that** the first end effector part (18) has an at least partially curved first bearing surface (80), and the second end effector part (20) has an at least partially curved second bearing surface (82), and the first and second bearing surfaces (80, 82) are each held slidably on the bearing element (22).

2. Endoscopic instrument according to claim 1, wherein the first thrust element (30) is articulatedly coupled to the first end effector part (18) by means of a first joint (44), and/or the second thrust element (32) is articulatedly coupled to the second end effector part (20) by means of a second joint (46), and/or the third thrust element (34) is articulatedly coupled to the first end effector part (18) and the second end effector part (20) by means of a third joint (48).

3. Endoscopic instrument according to any preceding claim, wherein the articulated coupling of the first thrust element (30) and the articulated coupling of the second thrust element (32) are configured in such a way that, during a displacement of the first thrust element (30) and/or of the second thrust element (32), the first end effector part (18) and the second end effector part (20) are pivotable relative to each other in a first pivot plane (HL) about a first pivot axis (50).

4. Endoscopic instrument according to any preceding claim, wherein the articulated coupling of the third thrust element (34) is configured in such a way that, during a displacement of the third thrust element (34), the first end effector part (18) and the second end effector part (20) are displaceable conjointly in a second pivot plane (LQ).

5. Endoscopic instrument according to any preceding claim, wherein the third thrust element (34) is coupled to the first end effector part (18) and the second end effector part (20) along a first pivot axis (50).

6. Endoscopic instrument according to one of claims 2 to 5, wherein the first joint (44) is configured in such a way that the first end effector part (18) can move relative to the first thrust element (30) about a first rotation axis (84) and about a further first rotation axis (86), wherein the first rotation axis (84) and the further first rotation axis (86) are at a first angle to each other, particularly at least approximately at a right angle.

7. Endoscopic instrument according to one of claims 3 to 6, wherein the first pivot axis (50) forms a longitudinal axis of a pivot pin (70) on which the first end effector part (18) and the second end effector part (20) are arranged, wherein in particular the pivot pin (70) protrudes from the bearing element (22) at a first side (72) and at a second side (74) lying opposite the first side.

8. Endoscopic instrument according to claim 7, wherein the pivot pin (70) is guided through a recess (104) of the bearing element (22), and/or the bearing element (22) is designed at least partially in the shape of a spherical segment.

9. Endoscopic instrument according to any preceding claim, wherein the first thrust element (30) and the second thrust element (32) lie in a first pivot plane (HL), and the third thrust element (34) lies in a second pivot plane (LQ) perpendicular to the first pivot plane (HL).

10. Endoscopic instrument according to any preceding claim, wherein the bearing element (22) is stationary in relation to the proximal end (12), and/or the bearing element (22) is arranged such that a displacement of one of the thrust elements (30, 32, 34) always leads to a displacement of the thrust element (30, 32, 34) relative to the bearing element (22).

11. Endoscopic instrument according to any preceding claim, wherein the first end effector part (18) has a first recess (92) and a further first recess (94), with which the first end effector part (18) is held on a pivot pin (70).

12. Endoscopic instrument according to any preceding claim, wherein the first bearing surface (80) has at least approximately the shape of a portion of a spherical surface, the portion resulting from a sphere being cut by two non-parallel planes which are at a plane angle to each other, the centre point of the sphere lying in both planes.

13. Endoscopic instrument according to any preceding claim, wherein all of the thrust elements (30, 32, 34) are arranged parallel to one another.

14. Endoscopic instrument according to any preceding claim, wherein the thrust elements (30, 32, 34) are designed as thrust rods, in particular designed to be entirely rectilinear.

15. Endoscopic instrument according to any preceding claim, wherein the first end effector part (18) is in the form of a first gripping forceps part and the second end effector part (20) is in the form of a second gripping forceps part, or wherein the first end effector part (18) is in the form of a first scissor part and the second end effector part (20) is in the form of a second scissor part.

## Revendications

1. Instrument endoscopique (10), avec
- une extrémité proximale (12),
- une extrémité distale (14),
- une direction longitudinale (L), le long de laquelle l'instrument endoscopique (10) s'étend de l'extrémité proximale (12) à l'extrémité distale (14),
- un effecteur d'extrémité (16) à l'extrémité distale (14), qui présente une première partie d'effecteur d'extrémité (18) et une deuxième partie d'effecteur d'extrémité (20),
- un élément de palier (22) à l'extrémité distale (14), sur lequel la première et la deuxième parties d'effecteur d'extrémité (18, 20) sont montées et sur lequel sont formées des parties d'une surface courbe,
- un premier élément de poussée (30),
- un deuxième élément de poussée (32), et
- un troisième élément de poussée (34),
dans lequel les éléments de poussée (30, 32, 34) s'étendent respectivement le long de la direction longitudinale (L) et sont déplaçables l'un par rapport à l'autre le long de la direction longitudinale (L),
dans lequel le premier élément de poussée (30) est couplé de façon articulée à la première partie d'effecteur d'extrémité (18), le deuxième élément de poussée (32) est couplé de façon articulée à la deuxième partie d'effecteur d'extrémité (20), le troisième élément de poussée (34) est couplé de façon articulée à la première partie d'effecteur d'extrémité (18) et à la deuxième partie d'effecteur d'extrémité (20),
**caractérisé en ce que** la première partie d'effecteur d'extrémité (18) présente une première surface d'appui (80) au moins localement courbe et la deuxième partie d'effecteur d'extrémité (20) présente une deuxième surface d'appui (82) au moins localement courbe et la première et la deuxième surfaces d'appui (80, 82) sont maintenues en mouvement glissant sur l'élément de palier (22).

2. Instrument endoscopique selon la revendication 1, dans lequel le premier élément de poussée (30) est couplé de façon articulée à la première partie d'effecteur d'extrémité (18) au moyen d'une première articulation (44) et/ou le deuxième élément de poussée (32) est couplé de façon articulée à la deuxième partie d'effecteur d'extrémité (20) au moyen d'une deuxième articulation (46) et/ou le troisième élément de poussée (34) est couplé de façon articulée à la première partie d'effecteur d'extrémité (18) et à la deuxième partie d'effecteur d'extrémité (20) au moyen d'une troisième articulation (48).

3. Instrument endoscopique selon l'une quelconque des revendications précédentes, dans lequel le couplage articulé du premier élément de poussée (30) et le couplage articulé du deuxième élément de poussée (32) sont configurés de telle manière que, lors d'un déplacement du premier élément de poussée (30) et/ou du deuxième élément de poussée (32), la première partie d'effecteur d'extrémité (18) et la deuxième partie d'effecteur d'extrémité (20) puissent pivoter l'une par rapport à l'autre dans un premier plan de pivotement (HL) autour d'un premier axe de pivotement (50).

4. Instrument endoscopique selon l'une quelconque des revendications précédentes, dans lequel le couplage articulé du troisième élément de poussée (34) est configuré de telle manière que, lors d'un déplacement du troisième élément de poussée (34), la première partie d'effecteur d'extrémité (18) et la deuxième partie d'effecteur d'extrémité (20) soient déplaçables en commun dans un deuxième plan de pivotement (LQ).

5. Instrument endoscopique selon l'une quelconque des revendications précédentes, dans lequel le troisième élément de poussée (34) est couplé le long d'un premier axe de pivotement (50) à la première partie d'effecteur d'extrémité (18) et à la deuxième partie d'effecteur d'extrémité (20).

6. Instrument endoscopique selon l'une quelconque des revendications 2 à 5, dans lequel la première articulation (44) est configurée de telle manière que la première partie d'effecteur d'extrémité (18) puisse se déplacer par rapport au premier élément de poussée (30) autour d'un premier axe de rotation (84) et autour d'un autre premier axe de rotation (86), dans lequel le premier axe de rotation (84) et l'autre premier axe de rotation (86) forment un angle l'un avec l'autre, en particulier au moins approximativement un angle droit.

7. Instrument endoscopique selon l'une quelconque des revendications 3 à 6, dans lequel le premier axe de pivotement (50) forme un axe longitudinal d'un arbre de pivotement (70), sur lequel la première partie d'effecteur d'extrémité (18) et la deuxième partie d'effecteur d'extrémité (20) sont montées, dans lequel en particulier l'arbre de pivotement (70) sort de l'élément de palier (22) sur un premier côté (72) et sur un deuxième côté (74) opposé au premier côté.

8. Instrument endoscopique selon la revendication 7, dans lequel l'arbre de pivotement (70) est guidé à travers une découpe (104) de l'élément de palier (22) et/ou l'élément de palier (22) est configuré au moins localement en forme de segment de sphère.

9. Instrument endoscopique selon l'une quelconque des revendications précédentes, dans lequel le premier élément de poussée (30) et le deuxième élément de poussée (32) sont situés dans un premier plan de pivotement (HL) et le troisième élément de poussée (34) est situé dans un deuxième plan de pivotement (LQ) perpendiculaire au premier plan de pivotement (HL).

10. Instrument endoscopique selon l'une quelconque des revendications précédentes, dans lequel l'élément de palier (22) est disposé de façon stationnaire par rapport à l'extrémité proximale (12) et/ou l'élément de palier (22) est disposé de telle manière qu'un déplacement d'un des éléments de poussée (30, 32, 34) conduise toujours à un déplacement de l'élément de poussée (30, 32, 34) par rapport à l'élément de palier (22).

11. Instrument endoscopique selon l'une quelconque des revendications précédentes, dans lequel la première partie d'effecteur d'extrémité (18) présente une première découpe (92) et une autre première découpe (94), avec lesquelles la première partie d'effecteur d'extrémité (18) est maintenue sur un arbre de pivotement (70).

12. Instrument endoscopique selon l'une quelconque des revendications précédentes, dans lequel la première surface d'appui (80) a au moins approximativement la forme d'une portion d'une surface sphérique, dans lequel la portion est obtenue par le fait qu'une sphère est coupée par deux plans non parallèles, qui forment entre eux un angle de plans, dans lequel le centre de la sphère se trouve dans les deux plans.

13. Instrument endoscopique selon l'une quelconque des revendications précédentes, dans lequel tous les éléments de poussée (30, 32, 34) sont disposés parallèlement l'un à l'autre.

14. Instrument endoscopique selon l'une quelconque des revendications précédentes, dans lequel les éléments de poussée (30, 32, 34) sont réalisés sous forme de bielles, et sont en particulier totalement rectilignes.

15. Instrument endoscopique selon l'une quelconque des revendications précédentes, dans lequel la première partie d'effecteur d'extrémité (18) est réalisée sous la forme d'une première partie de pince et la deuxième partie d'effecteur d'extrémité (20) est réalisée sous la forme d'une deuxième partie de pince ou dans lequel la première partie d'effecteur d'extrémité (18) est réalisée sous la forme d'une première partie de ciseaux et la deuxième partie d'effecteur d'extrémité (20) est réalisée sous la forme d'une deuxième partie de ciseaux.
